# EUROPEAN PATENT SPECIFICATION

(11) **EP 2 452 630 B1**
(45) Date of publication and mention of the grant of the patent: **21.09.2016**
(21) Application number: 11188367.4
(22) Date of filing: 09.11.2011
(51) Int. Cl.: A61B 17/00

(54) **Surgical instrument including accessory powering feature**
OP-Instrument einschließlich Zubehörstromversorgungseigenschaft
Instrument chirurgical incluant une fonction d'alimentation d'accessoires

(30) Priority: 10.11.2010 US 412140 P; 26.10.2011 US 201113281572
(43) Date of publication of application: 16.05.2012
(60) Divisional application: 16183302.5
(73) Proprietor: Covidien LP, Mansfield, MA 02048 (US)
(72) Inventor: Viola, Frank, Sandy Hook, Connecticut 06482 (US)
(74) Representative: Soames, Candida Jane

(56) References cited:
- EP-A1- 1 913 864
- WO-A2-2004/035106
- WO-A2-2009/134634
- US-A- 4 542 741
- US-A- 5 785 408
- US-A- 5 951 142
- US-A- 6 029 303
- US-A1- 2006 291 195
- US-A1- 2009 157 092

## Description

### Technical field

This application relates to surgical instruments and more particularly, to energy sources for use with surgical instrument accessories.

### Background of Related Art

A typical surgery employs a plurality of different surgical instruments and accessory devices for use with the various surgical instruments. When attaching accessory devices, e.g., illumination devices or cameras, there is often a need to satisfy the energy needs of the accessory device. While self contained energy sources like batteries are often utilized, they take up valuable space in the accessory device, thereby increasing the size of the accessory which is disadvantageous in minimally invasive surgery. Additionally, such energy sources often have limited energy storage capacity. As such, removal or repositioning of the accessory may be necessary to change a battery or other energy storage device, which, if required during surgery or other medical procedure, can reduce efficiency and increase the time and complexity of the surgery.

EP 1913864 A1 discloses a self-contained illuminator, attachable to a medical instrument, comprising an instrument coupling head having a lighting element at a first end and at a second end a first electrical connector releasably attachable to a second compatible electrical connector of a power source. Embodiments show the illuminator attached to and running along the exterior of a tooth scaler and a mouth mirror.

US 2006/029195 A1 discloses a self-powered lighting system that can be readily attached to a known surgical tool to provide prolonged, clear, consistent lighting at a surgical site, and covey heat energy away from the surgical site, comprising a self-contained power unit, a connection element, and a light emitting element. The light emitting element, connecting element, and power unit being operably connected to one another, and the power unit, connection element, and light-emitting element having means to attach to a surgical tool.

US 6,029,303 describes a toothbrush comprising an electronic device operably connected to a power source. The electronic device is activated by use of the toothbrush and is programmed to output a detectable output sequence to notify the user that a particular condition has been met.

WO 2004/035106 is concerned with an intubation tool comprising an imaging unit and may also include a power source for powering components of the imaging unit.

WO 2009/134634 A2 describes a disposable cannula comprising illumination and/or vision systems that may be powered by flexible electrical lines running along the body of the cannula coupling with an external control and display unit. The cannula is configured to allow a surgical instrument to be inserted therethrough.

US 5,951,142 discloses an adjustable illuminating apparatus comprising a shaft and a light source. The light source is mounted on the shaft. The light-source comprises an annular hood that may be extended or retracted to focus or diverge the light emitted, respectively. A battery may be included in the apparatus, electronically connected to the light source via a conducting wire connected to the light source and an iron wire connected to the metallic shaft and light source housing.

US 4,542,741 describes a surgical instrument comprising an instrument head, a handle, and a light source. In embodiments, the light source is contained within a separate battery pack that may be attached to the surgical instrument. The light source and battery packs are self-contained and not electronically coupled to the handle.

US 5,785,408 is directed to an illuminating device for engaging onto a tool. Tools mentioned as suitable are a wrench or a screw driver. The illuminating device comprises a housing, light bulb, means for energising the light bulb, and means for attaching the housing to the tool. The energy device, embodied by batteries, is secured in the housing.

### SUMMARY

Accordingly, the present disclosure in one aspect is directed to a surgical instrument system that includes a surgical instrument, an accessory, and an energy device. The surgical instrument includes a housing, a shaft extending from the housing, and a tool assembly operably coupled to the shaft. The accessory is operably couplable to the surgical instrument. The energy device is operably coupled to the accessory when the accessory is operably coupled to the surgical instrument such that the energy device powers the accessory.

An instrument powering device can be provided in some embodiments which is operably coupled to one or more of the housing, the shaft, and the tool assembly. The instrument powering device can be configured and dimensioned to power the surgical instrument. In some embodiments, the instrument powering device is configured and dimensioned to solely power the tool assembly.

The surgical instrument may include one or more contacts. The one or more contacts may be positioned on the shaft. In some embodiments, one or more contacts of the surgical instrument include one or more slip rings electrically coupled to the energy device.

The accessory may include one or more of a camera and an illumination device. The accessory in some embodiments defines a channel therethrough and one or more contacts. The channel in some embodiments is configured and dimensioned to accommodate at least a portion of the shaft when the accessory is operably coupled to the surgical instrument. The one or more contacts may be positioned within the channel such that when the accessory is positioned on the shaft, where one or more contacts are positioned on the shaft, two or more contacts are engaged so that the accessory is electrically coupled to the energy device.

In some embodiments, the energy device is operably coupled to one or more of the contacts of the accessory, and the energy device transmits power to the accessory when the one or more contacts of the accessory and the one or more contacts of the surgical instrument are in contact. In some embodiments, the energy device includes an energy storage device that is removably positionable within the surgical instrument. The energy storage device may be positionable within the housing of the surgical instrument. In some embodiments, the energy storage device is a battery.

In another aspect, the present disclosure provides a surgical instrument system comprising a surgical instrument having a housing, a shaft extending from the housing, a tool assembly operably coupled to the shaft, and an instrument powering device operably coupled to at least one of the housing, the shaft, and the tool assembly. The instrument powering device is configured and dimensioned to solely power the surgical instrument. An accessory is operably couplable to the surgical instrument, and operably coupled to the instrument powering device when the accessory is operably coupled to the surgical instrument such that the instrument powering device powers the accessory.

In some embodiments, the accessory includes at least one contact and the instrument includes at least one contact, and the at least one contact of the accessory and the at least one contact of the surgical instrument are in contact when the accessory is coupled to the instrument. In some embodiments, the at least one contact of the surgical instrument is positioned on the shaft of the surgical instrument.

In another aspect, the present disclosure provides an accessory for use with a surgical instrument. The accessory is adapted and dimensioned to perform an operation different from, or supplemental to, the operations performed by the surgical instrument. The surgical instrument has a first energy source and at least one electrical contact. The first energy source provides power to the accessory. The accessory includes at least one electrical contact adapted and dimensioned to engage the at least one electrical contact of the surgical instrument in order to electrically couple the accessory to the first energy source.

A second energy source can be provided to power the surgical instrument. In some embodiments, the second energy source provides sole power to the surgical instrument. In some embodiments, the first energy source provides power to solely power the accessory.

### BRIEF DESCRIPTION OF THE DRAWINGS

Various embodiments of the present disclosure are described herein with reference to the drawings wherein:
Fig. 1 is a perspective view of one embodiment of a surgical instrument system in accordance with the present disclosure;
Fig. 1A is a perspective view of another embodiment of the surgical instrument system; and
Fig. 2 is a partial perspective view, with parts separated, of a distal portion of the surgical instrument system of Fig. 1.

### DETAILED DESCRIPTION

Embodiments of the presently disclosed surgical instrument are described in detail with reference to the drawings, wherein like reference numerals designate similar or identical elements in each of the several views. In the drawings and the description that follows, the term "proximal" refers to the end of the surgical instrument that is closer to the user, whereas the term "distal" refers to the end of the surgical instrument that is further from the user.

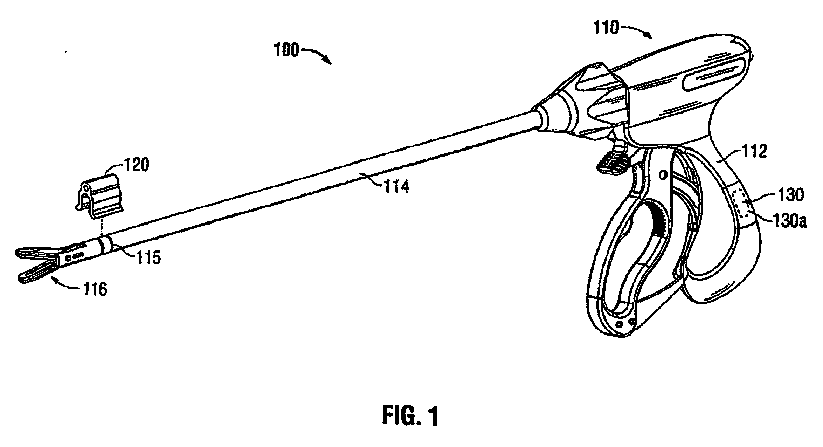

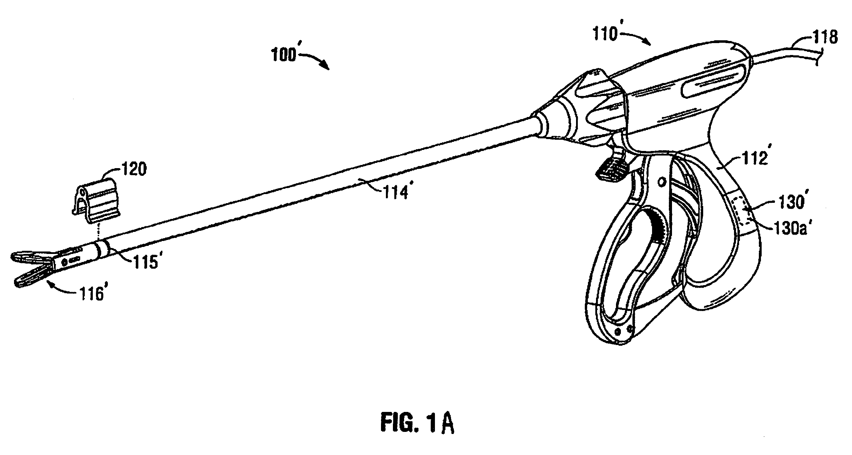

Referring now to the drawings, Fig. 1 illustrates one embodiment of a surgical instrument system 100. The surgical instrument illustrated is a surgical grasper, it being understood that other surgical instruments can be utilized such as endoscopic scissors, clip appliers, surgical staplers, etc. The surgical instrument system 100 includes a surgical instrument 110, an accessory 120, and an energy device 130. The accessory 130 can be powered by external corded power supplies or internal power supplies that are wired internally to contacts on the instrument as described below.

The surgical instrument 110 includes a housing 112, a shaft 114 extending distally from the housing 112, and a tool assembly 116 at a distal end portion of the shaft 114.

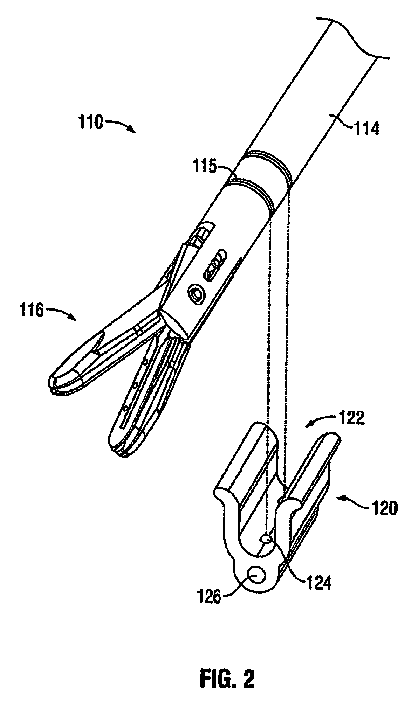

The surgical instrument 110 includes one or more contacts 115. In the illustrated embodiment, two ring contacts 115 are shown by way of example. The contacts 115 may be positioned on the shaft 114. The contacts 115 may include contact buttons, slip rings, or any other suitable connection. The contacts 115 are electrically coupled to the energy storage device 130 (e.g., via one or more wires extending therebetween (through the shaft 114) and/or a wireless connection) such that the energy device 130 transmits power to the contacts 115. The contacts 115 are engaged by the contacts 124 of the accessory 120 (Fig. 2). In this manner, the power is transmitted to the accessory 120 from the energy device 130 through the contacts 115, 124.

Referring now to Fig. 2, the accessory 120 is mountable, and preferably removably mountable, to the surgical instrument 110. The accessory 120 defines a channel 122 therethrough and includes one or more contacts 124. The channel 122 is configured and dimensioned to accommodate at least a portion of the shaft 114 when the accessory 120 is selectively operably coupled to the surgical instrument 110. Thus, the accessory can frictionally engage the shaft 112. Alternatively, the accessory can be attached to the shaft by a snap fit, interlocking structure, or by other methods. The contacts 124 may be positioned within the channel 122 such that when the accessory 120 is positioned on the shaft 114 adjacent contacts 115, two or more contacts (i.e., at least one contact 115 and at least one contact 124) are engaged so that the accessory 120 is electrically coupled to the energy device 130. In particular, the contacts 124 may be operably coupled to the energy device 130 via any suitable electrical or electromechanical, features. For example, these features, which may include wired or wireless connections, may have inductive components, capacitive components, resistive components, switching components, etc. that facilitate the connection between the accessory 120 and the surgical instrument 110 so that the accessory 120 can be powered by the energy device 130. Furthermore, the contacts 124 may include contact buttons, slip rings, or any other suitable connection components. The accessory 120 may include one or more powered devices 126 including cameras, illumination devices, or any other suitable powered devices that could assist the clinician in performing a medical procedure. Consequently, the accessory device can be more compact, i.e. have a reduced profile, as the energy storage device is part of the instrument and not the accessory.

The energy device 130 is operably or electrically coupled with the accessory 120 when the accessory 120 is operably coupled to the surgical instrument 110 such that the energy device 130 powers the accessory 120. In some embodiments, the energy device 130 solely powers the accessory. The energy device 130 is operably or electrically coupled to one or more of the contacts 124 of the accessory 120. The energy device 130 transmits power to the accessory 120 when the one or more contacts 124 of the accessory 120 and the one or more contacts 115 of the surgical instrument 110 are in contact. The energy device 130 includes an energy storage device 130a that may be a battery, rechargeable battery, or any other suitable self-contained electrical energy source. The energy storage device 130a may be removably positionable within the surgical instrument 110 so it can be removed and recharged or removed and replaced with another energy storage device. As seen in Fig. 1, the energy storage device 130a may be positionable within the housing 112, and more particularly in the handle, of the surgical instrument 110. Positioning in other locations in the instrument are also contemplated, as well as positioning outside the instrument.

Although electrical energy sources for the accessory are disclosed herein, other energy sources are also contemplated such as fluid power sources.

In the alternate embodiment of Fig. 1A, the surgical instrument 100' includes an instrument powering device 118 operably coupled to one or more of the housing 112', the shaft 114', and the tool assembly 116'. The instrument powering device 118 is configured and dimensioned to power the surgical instrument 110'. The instrument powering device 118 may be an electrical connection adapted to engage an outlet and/or a generator or any other suitable power source for the reception and/or passage of electrical or electromechanical energy therethrough. In some embodiments, the instrument powering device 118 is configured and dimensioned to solely power the surgical instrument. In some embodiments, it solely powers the tool assembly 116'.

The instrument 100' includes an energy device 130', similar to energy device 130 of Figure 1, positioned in handle 112'. The energy device 130' can alternatively be positioned in other locations in the instrument. In some embodiments, the powering device 118 supplies power to the surgical instrument (e.g. jaws 116') and the energy storage device supplies power to the accessory. In other embodiments, the powering device powers the surgical instrument and the accessory, while the energy storage device provides backup power to the accessory. The powering device can be the sole power source for the instrument.

Instrument 100' has contacts 115' on shaft 114' extending from housing 110' identical to contacts 115 of Figure 1 to provide electrical contact with accessory 120 of Figure 2.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A surgical instrument system (100), comprising:
a surgical instrument (110) including:
a housing (112);
a shaft (114) extending from the housing (112); and
a tool assembly (116) operably coupled to the shaft (114);
an accessory (120) operably couplable to the surgical instrument (110); and
an energy device (130) operably coupled to the accessory (120) when the accessory (120) is operably coupled to the surgical instrument (110) such that the energy device (130) powers the accessory (120) **characterised in that**:
the accessory (120) includes at least one contact (124) and the surgical instrument (110) includes at least one contact (115), and the energy device (130) is operably coupled to at least one of the contacts (115, 124),
wherein the energy device (130) transmits power to the accessory (120) when the at least one contact (124) of the accessory (120) and the at least one contact (115) of the surgical instrument (110) are in contact.

2. The surgical instrument system (100) according to claim 1, wherein at least one contact (115) of the surgical instrument (110) includes at least one slip ring, the at least one slip ring being electrically coupled to the energy device (130).

3. The surgical instrument system (100) according to claim 1 or claim 2, wherein the accessory (120) defines a channel (122) therethrough, the channel (122) configured and dimensioned to accommodate at least a portion of the shaft (114) when the accessory (120) is operably coupled to the surgical instrument (110).

4. The surgical instrument system (100) according to any of claims 1-3, wherein the at least one contact (115) of the surgical instrument (110) is positioned on the shaft (114).

5. The surgical instrument system (100) according to any of claims 1-4, wherein the at least one contact (124) of the accessory (120) is positioned within the channel (122) such that when the accessory (120) is positioned on the shaft (114), at least one contact of the accessory and one contact of the surgical instrument (115, 124) are engaged so that the accessory (120) is electrically coupled to the energy device (130).

6. The surgical instrument system (100) according to any of claims 1-5, wherein the energy device (130) includes an energy storage device (130a) removably positionable within the surgical instrument (110).

7. The surgical instrument system (100) according to claim 6, wherein the energy storage device (130a) is positionable within the housing (112) of the surgical instrument (110).

8. The surgical instrument system (100) according to any of claims 1-7, wherein the accessory (120) includes at least one of a camera and an illumination device.

9. The surgical instrument system (100) according to any of claims 1-8, further comprising an instrument powering device (118) operably coupled to at least one of the housing (112), the shaft (114), and the tool assembly (116), the instrument powering device (118) powering the accessory (120) and the instrument (110).

10. The surgical instrument system (100) according to any of claims 1-8, further comprising an instrument powering device (118) operably coupled to at least one of the housing (112), the shaft (114), and the tool assembly (116), the instrument powering device (118) configured and dimensioned to solely power the surgical instrument (110).

11. The surgical instrument system (100) according to claims 9 or 10, wherein the instrument powering device (118) is operably coupled to an external generator.

12. The surgical instrument system (100) according to any of claims 1-11, wherein the accessory (120) is adapted and dimensioned to perform an operation different from, or supplemental to, operations performed by the surgical instrument (110).

13. The surgical instrument system (100) according to any of claims 1-12, wherein the energy storage device (130a) includes a battery.

## Patentansprüche

1. Chirurgisches Instrumentensystem (100), umfassend:
- ein chirurgisches Instrument (110), enthaltend:
* ein Gehäuse (112);
* einen Schaft (114), der sich aus dem Gehäuse (112) erstreckt; und
* eine Instrumentenmontage (116), betriebsbereit an den Schaft (114) koppelbar;
- ein Zubehörteil (120), betriebsbereit an das chirurgische Instrument (110) koppelbar; und
- eine Energievorrichtung (130), betriebsbereit an das Zubehörteil (120) koppelbar, wenn das Zubehörteil (120) betriebsbereit an das chirurgische Instrument (110) gekoppelt ist, derart, dass die Energievorrichtung (130) das Zubehörteil (120) mit Strom versorgt, **dadurch gekennzeichnet, dass** besagtes Zubehörteil (120) mindestens einen Kontakt (124) aufweist und dass besagtes chirurgisches Instrument (110) mindestens einen Kontakt (115) aufweist, und dass die Energievorrichtung (130) betriebsbereit an mindestens einen der Kontakte (115, 124) gekoppelt ist, wobei die Energievorrichtung (130) Strom an das Zubehörteil (120) überträgt, wenn der mindestens eine Kontakt (124) des Zubehörteils (120) und der mindestens eine Kontakt (115) des chirurgischen Instruments (110) in Kontakt stehen.

2. Chirurgisches Instrumentensystem (100) gemäß Anspruch 1, worin mindestens ein Kontakt (115) des chirurgischen Instruments (110) mindestens einen Schleifring enthält, wobei der mindestens eine Schleifring elektrisch an die Energievorrichtung (130) gekoppelt ist.

3. Chirurgisches Instrumentensystem (100) gemäß Anspruch 1 oder Anspruch 2, worin das Zubehörteil (120) einen durchführenden Kanal (122) definiert, wobei der Kanal (122) konfiguriert und dimensioniert ist, um mindestens einen Abschnitt des Schafts (114) aufzunehmen, wenn das Zubehörteil (120) betriebsbereit an das chirurgische Instrument (110) gekoppelt ist.

4. Chirurgisches Instrumentensystem (100) gemäß einem der Ansprüche 1 bis 3, worin der mindestens eine Kontakt (115) des chirurgischen Instruments (110) auf dem Schaft (114) positioniert ist.

5. Chirurgisches Instrumentensystem (100) gemäß einem der Ansprüche 1 bis 4, worin der mindestens eine Kontakt (124) des Zubehörteils (120) im Kanal (122) positioniert ist, derart, dass wenn das Zubehörteil (120) auf dem Schaft (114) positioniert ist, mindestens ein Kontakt des Zubehörteils und mindestens ein Kontakt des chirurgischen Instruments (115, 124) eingreifen, sodass das Zubehörteil (120) elektrisch an die Energievorrichtung (130) gekoppelt ist.

6. Chirurgisches Instrumentensystem (100) gemäß einem der Ansprüche 1 bis 5, worin die Energievorrichtung (130) einen Energiespeicher (130a) enthält, der entfernbar innerhalb des chirurgischen Instruments (110) positionierbar ist.

7. Chirurgisches Instrumentensystem (100) gemäß Anspruch 6, worin der Energiespeicher (130a) innerhalb des Gehäuses (112) des chirurgischen Instruments (110) positionierbar ist.

8. Chirurgisches Instrumentensystem (100) gemäß einem der Ansprüche 1 bis 7, worin das Zubehörteil (120) mindestens eine Kamera und eine Beleuchtungsvorrichtung enthält.

9. Chirurgisches Instrumentensystem (100) gemäß einem der Ansprüche 1 bis 8, zusätzlich umfassend: eine Antriebsvorrichtung für das Instrument (118), betriebsbereit mindestens an das Gehäuse (112), den Schaft (114) und die Instrumentenmontage (116) gekoppelt, wobei die Antriebsvorrichtung für das Instrument (118) das Zubehörteil (120) und das Instrument (110) antreibt.

10. Chirurgisches Instrumentensystem (100) gemäß einem der Ansprüche 1 bis 8, zusätzlich umfassend: eine Antriebsvorrichtung für das Instrument (118), betriebsbereit mindestens an das Gehäuse (112), den Schaft (114) und die Instrumentenmontage (116) gekoppelt, wobei die Antriebsvorrichtung für das Instrument (118), wobei die Antriebsvorrichtung für das Instrument (118) konfiguriert und dimensioniert ist, um einzig und allein das chirurgische Instrument (110) anzutreiben.

11. Chirurgisches Instrumentensystem (100) gemäß einem der Ansprüche 9 oder 10, worin die Antriebsvorrichtung für das Instrument (118) betriebsbereit an einen externen Generator gekoppelt ist.

12. Chirurgisches Instrumentensystem (100) gemäß einem der Ansprüche 1 bis 11, worin das Zubehörteil (120) geeignet und dimensioniert ist, um eine unterschiedliche oder zusätzliche Aktion durchzuführen, als die vom chirurgischen Instrument (110) durchgeführten Aktionen.

13. Chirurgisches Instrumentensystem (100) gemäß einem der Ansprüche 1 bis 12, worin der Energiespeicher (130a) eine Batterie enthält.

## Revendications

1. Système d'instrument chirurgical (100), comprenant :
un instrument chirurgical (110) comprenant :
un logement (112) ;
un arbre (114) s'étendant à partir du logement (112) ; et
un ensemble outil (116) couplé de manière opérationnelle à l'arbre (114) ;
un accessoire (120) pouvant être couplé de manière opérationnelle à l'instrument chirurgical (110) ; et
un dispositif d'énergie (130) couplé de manière opérationnelle à l'accessoire (120) quand l'accessoire (120) est couplé de manière opérationnelle à l'instrument chirurgical (110) de sorte que le dispositif d'énergie (130) alimente l'accessoire (120) **caractérisé en ce que** :
l'accessoire (120) inclut au moins un contact (124) et l'instrument chirurgical (110) inclut au moins un contact (115), et le dispositif d'énergie (130) est couplé de manière opérationnelle à au moins un des contacts (115, 124),
dans lequel le dispositif d'énergie (130) transmet de la puissance à l'accessoire (120) quand l'au moins un contact (124) de l'accessoire (120) et l'au moins un contact (115) de l'instrument chirurgical (110) sont en contact.

2. Système d'instrument chirurgical (100) selon la revendication 1, dans lequel au moins un contact (115) de l'instrument chirurgical (110) inclut au moins une bague collectrice, l'au moins une bague collectrice étant électriquement couplée au dispositif d'énergie (130).

3. Système d'instrument chirurgical (100) selon la revendication 1 ou la revendication 2, dans lequel l'accessoire (120) définit un canal (122) à travers ce dernier, le canal (122) étant configuré et dimensionné pour loger au moins une partie de l'arbre (114) lorsque l'accessoire (120) est couplé de manière opérationnelle à l'instrument chirurgical (110).

4. Système d'instrument chirurgical (100) selon l'une quelconque des revendications 1 à 3, dans lequel l'au moins un contact (115) de l'instrument chirurgical (110) est positionné sur l'arbre (114).

5. Système d'instrument chirurgical (100) selon l'une quelconque des revendications 1 à 4, dans lequel l'au moins un contact (124) de l'accessoire (120) est positionné à l'intérieur du canal (122) de sorte que lorsque l'accessoire (120) est positionné sur l'arbre (114), au moins un contact de l'accessoire et un contact de l'instrument chirurgical (115, 124) sont en prise de sorte que l'accessoire (120) est électriquement couplé au dispositif d'énergie (130).

6. Système d'instrument chirurgical (100) selon l'une quelconque des revendications 1 à 5, dans lequel le dispositif d'énergie (130) inclut un dispositif de stockage d'énergie (130a) pouvant être positionné de façon amovible à l'intérieur de l'instrument chirurgical (110).

7. Système d'instrument chirurgical (100) selon la revendication 6, dans lequel le dispositif de stockage d'énergie (130a) peut être positionné à l'intérieur du logement (112) de l'instrument chirurgical (110).

8. Système d'instrument chirurgical (100) selon l'une quelconque des revendications 1 à 7, dans lequel l'accessoire (120) inclut au moins un d'un appareil de prise de vues et d'un dispositif d'éclairage.

9. Système d'instrument chirurgical (100) selon l'une quelconque des revendications 1 à 8, comprenant en outre un dispositif d'alimentation d'instrument (118) couplé de manière opérationnelle à au moins un du logement (112), de l'arbre (114) et de l'ensemble outil (116), le dispositif d'alimentation d'instrument (118) alimentant l'accessoire (120) et l'instrument (110).

10. Système d'instrument chirurgical (100) selon l'une quelconque des revendications 1 à 8, comprenant en outre un dispositif d'alimentation d'instrument (118) couplé de manière opérationnelle à au moins un du logement (112), de l'arbre (114) et de l'ensemble outil (116), le dispositif d'alimentation d'instrument (118) étant configuré et dimensionné pour alimenter seulement l'instrument chirurgical (110).

11. Système d'instrument chirurgical (100) selon les revendications 9 ou 10, dans lequel le dispositif d'alimentation d'instrument (118) est couplé de manière opérationnelle à un générateur externe.

12. Système d'instrument chirurgical (100) selon l'une quelconque des revendications 1 à 11, dans lequel l'accessoire (120) est conçu et dimensionné pour effectuer une opération différente, ou supplémentaire à, des opérations effectuées par l'instrument chirurgical (110).

13. Système d'instrument chirurgical (100) selon l'une quelconque des revendications 1 à 12, dans lequel le dispositif de stockage d'énergie (130a) inclut une batterie.
